# DEMANDE DE BREVET EUROPEEN

(11) **EP 2 832 721 A1**
(43) Date de publication de la demande: **04.02.2015**
(21) Numéro de dépôt: 14178246.6
(22) Date de dépôt: 24.07.2014
(51) Int. Cl.: C07C 407/00, C07C 409/26, A61L 2/18

(54) **Composition a base de tetraacetylethylenediamine pour la generation d'acide peracetique**

(30) Priorité: 02.08.2013 FR 1357703
(71) Demandeur: CAIR L.G.L., 69380 Civrieux D'Azergues (FR)
(72) Inventeur: Lorenzini, Giorgio, 37045 LEGNAGO (VR) (IT)
(74) Mandataire: Cabinet Laurent & Charras

(57) **Abrégé**

Composition se présentant sous la forme de poudre, comprenant, en poids sec par rapport au poids de la composition :
- 58.5 à 73.5 % d'au moins un sel de monopersulfate ;
- 25 à 40 % de tétra acétyléthylènediamine ;
- 1.5 à 2.5 % d'au moins un sel d'acétate.

## Description

### DOMAINE DE L'INVENTION

L'invention concerne une composition apte à générer de l'acide peracétique lors de sa mise en solution dans de l'eau.

Le domaine d'utilisation de la présente invention s'inscrit dans la biologie et notamment dans les applications cherchant un effet fongicide et/ou biocide notamment sur des cellules vivantes ou des virus. Il peut s'agir plus particulièrement de la désinfection d'instruments de dialyse.

### ETAT ANTERIEUR DE LA TECHNIQUE

L'acide peracétique, CH₃C(=O)-O-O-H, ou acide peroxyacétique est couramment utilisé dans l'industrie eu égard à ses propriétés désinfectantes et stérilisantes. En effet, il s'agit d'un biocide dont l'efficacité outrepasse généralement celle du peroxyde d'hydrogène.

Toutefois, en raison de son instabilité, cet acide ne peut pas être stocké à l'état pur. En outre, il peut être décomposé en présence d'ions métalliques, ou en fonction de la température de l'eau de dilution. Il convient donc de préparer soit des compositions comprenant des stabilisants, soit des compositions permettant de générer l'acide peracétique dans des conditions prédéterminées.

Dans tous les cas, les solutions contenant de l'acide peracétique présentent donc une durée d'utilisation limitée.

L'acide peracétique peut être préparé à partir du mélange acide acétique / peroxyde d'hydrogène selon l'équilibre suivant :

CH₃C(=O)-O-H + H₂O₂ ↔ CH₃C(=O)-O-O-H + H₂O

Les solutions aqueuses d'acide peracétique issues de cette réaction comprennent donc un mélange acide peracétique/acide acétique/peroxyde d'hydrogène dont l'équilibre dépend notamment de la dégradation de l'acide peracétique en acide acétique en présence d'eau.

L'équilibre de cette réaction dépend également de la réaction secondaire de décomposition du peroxyde d'hydrogène en eau et oxygène :

2 H₂O₂ → 2H₂O + O₂

De manière générale, la décomposition d'une solution de peroxyde d'hydrogène est fonction de la température, de la concentration de la solution mais aussi de l'éventuelle présence d'impuretés et de stabilisants.

Le peroxyde d'hydrogène ainsi consommé ne peut donc pas participer à la génération d'acide peracétique.

Une autre alternative consiste à préparer l'acide peracétique par perhydrolyse c'est-à-dire en présence d'un radical acétyl, CH₃C(=O)-O^{·}, et d'ions perhydroxyl H-O-O⁻.

Un autre mode d'obtention de l'acide peracétique consiste à faire réagir la tétraacétyléthylènediamine (TAED) et le peroxyde d'hydrogène selon la réaction suivante :

(CH₃C(=O))₂-N-CH₂-CH₂-N-(C(=O)CH₃)₂ + 2 H₂O₂ → (CH₃C(=O))-NH-CH₂CH₂-NH-(C(=O)CH₃) + 2 CH₃CO₃H

Deux équivalents d'acide peracétique et un équivalent de diacétyléthylènediamine (DAED) sont ainsi générés pour chaque molécule de TAED de départ.

Afin notamment d'obtenir des solutions dont la concentration en acide peracétique peut être prédéterminée malgré les réactions de dégradation, des compositions se présentant sous la forme de poudre, et plus stables ont été développées. Elles sont généralement à base de TAED et de percarbonate de sodium, Na₂CO₃.1.5 H₂O₂. La mise en solution de ces compositions permet de générer le peroxyde d'hydrogène in situ et ainsi d'obtenir de l'acide peracétique à partir du TAED.

Bien qu'apparemment satisfaisantes, les compositions solides à base de TAED et de percarbonate de sodium présentent toutefois l'inconvénient de ne pas pouvoir être prêtes à l'emploi immédiatement. En effet, le peroxyde d'hydrogène doit être généré, avant de pouvoir réagir avec le TAED flottant à la surface de l'eau.

Etant donné que le TAED n'est pas totalement soluble dans l'eau, la cinétique relative à sa réactivité avec le peroxyde d'hydrogène est d'autant plus lente. Il s'agit donc, dans un premier temps, de générer rapidement du peroxyde d'hydrogène et, dans un deuxième temps, de favoriser la réaction entre le peroxyde d'hydrogène généré et le TAED flottant à la surface de l'eau.

Toutefois, aucune des compositions de l'art antérieur ne permet d'améliorer la cinétique de formation de l'acide peracétique. En effet, eu égard à l'instabilité de l'acide peracétique généré et des réactions parasites telles que la décomposition du peroxyde d'hydrogène, il est crucial de disposer d'une composition permettant de générer le plus rapidement possible une solution aqueuse d'acide peracétique, à une concentration prédéterminée.

En outre, l'acide peracétique se dégradant au cours du temps, sa génération rapide est généralement incompatible avec la stabilité dans le temps de la solution le contenant. A cet égard, l'activité biocide et/ou fongicide de la solution peut être rapidement dégradée et donc diminuer le temps d'utilisation de la composition.

Le Demandeur a mis au point une composition solide remédiant aux écueils de l'art antérieur en permettant notamment la génération rapide d'acide peracétique en solution aqueuse, et en assurant une concentration en acide peracétique satisfaisante plusieurs jours après sa génération.

### EXPOSE DE L'INVENTION

Eu égard à l'instabilité de l'acide peracétique, il existe une demande forte concernant les compositions permettant sa génération quasi immédiate à partir d'une formulation solide.

Le Demandeur a mis au point une composition comprenant du monopersulfate ainsi que du tétra acétyléthylènediamine (TAED). Eu égard aux quantités relatives des constituants mis en oeuvre, cette composition permet, dans un temps court, de générer une solution d'acide peracétique par addition d'eau juste avant utilisation. La concentration en acide peracétique peut ainsi être prédéterminée.

La composition et la solution aqueuse la comprenant sont biodégradables, l'acide peracétique se décomposant notamment en acide acétique selon la durée et la température de stockage.

Plus précisément, la présente invention concerne une composition se présentant sous la forme de poudre, comprenant, en poids sec par rapport au poids de la composition :
- 58.5 à 73.5 % d'au moins un sel de monopersulfate, avantageusement de 62 à 68 % ;
- 25 à 40 % de tétra acétyléthylènediamine, avantageusement de 30 à 35 % ;
- 1.5 à 2.5 % d'au moins un sel d'acétate, avantageusement de l'ordre de 2%.

Par poudre, on entend également une poudre comprenant des cristaux.

En outre, la composition objet de l'invention est hygroscopique. La présence d'eau dans la composition étant généralement nocive à sa stabilité dans le temps, elle est donc avantageusement conservée à l'abri de l'humidité mais aussi à la température ambiante.

Elle est toutefois de manière avantageuse anhydre.

Par monopersulfate, on entend les ions HSO₅⁻.

Le sel de monopersulfate peut être choisi dans le groupe comprenant les sels d'ammonium, d'alcalin, d'alcalino-terreux de monopersulfate, et leurs mélanges. Il s'agit préférentiellement du monopersulfate de potassium, de formule 2 KHSO₅ KHSO₄ K₂SO₄ et dont le numéro CAS est 70693-62-8.

De la même manière, le sel d'acétate peut être choisi dans le groupe comprenant les sels d'ammonium, d'alcalin, d'alcalino-terreux d'acétate, et leurs mélanges. Il s'agit préférentiellement de l'acétate de sodium, CH₃C(=O)ONa.

Selon un mode de réalisation particulièrement préféré, la composition objet de la présente invention comprend, en poids sec :
- 65.3 % de potassium monopersulfate ;
- 32.7 % de tétra acétyléthylènediamine ;
- 2 % de sodium acétate.

La composition objet de l'invention peut être conditionnée sous la forme de pastille (tablette, pilule, comprimé) dont le poids est adapté aux containers de l'utilisateur. Dans la pratique, ces containers présentent un volume de l'ordre de 5 litres. Ainsi l'utilisateur pourra aisément doser la composition en ajoutant une ou plusieurs tablettes en fonction du volume du container et de la concentration d'acide peracétique voulue.

La présente invention concerne également un procédé pour préparer une solution aqueuse biocide, selon lequel on mélange, à une température comprise entre 4 et 40°C, de 110 à 120 parts en poids de la composition décrite ci-dessus, avec 4500 à 5500 parts en poids d'eau.

La génération d'acide peracétique selon ce procédé est avantageusement réalisée dans une durée de 2 à 3 minutes, à la température ambiante.

Par génération d'acide peracétique, on entend l'obtention d'une solution comprenant entre 0.075 et 0.10 mol.L⁻¹ d'acide peracétique, c'est-à-dire une concentration suffisante pour conférer à la solution des propriétés bactéricide et fongicide.

L'homme du métier saura contrôler l'activité de la solution dans le temps, c'est-à-dire la concentration en acide peracétique en solution.

La solution aqueuse biocide et/ou fongicide et/ou virucide susceptible d'être obtenue selon le procédé décrit ci-avant entre également dans le cadre de la présente invention.

En outre, la présente invention concerne également l'utilisation de la solution aqueuse d'acide peracétique dans le domaine de la biologie et notamment dans les applications cherchant un effet biocide sur des cellules vivantes ou des virus. Cette solution peut ainsi permettre d'assainir un milieu par élimination des micro-organismes.

Cette solution aqueuse présente des propriétés biocides provenant de la génération de l'acide peracétique qui peut agir sur les membranes lipoprotéiques des cellules, sur le noyau et sur les autres composants essentiels à la vie de la cellule. En outre, l'acide peracétique permet de modifier la fonction osmotique de la membrane de la cellule, et ainsi de détruire la cellule.

La présente invention concerne également l'utilisation de cette solution biocide et/ou fongicide et/ou virucide comme agent désinfectant dans le domaine de la dialyse, notamment pour la désinfection d'instruments de dialyse. Il peut notamment s'agir du circuit de la machine à dialyse dans lequel circule le fluide de dialyse.

L'invention et les avantages qui en découlent ressortiront mieux des exemples suivants donnés afin d'illustrer l'invention et non de manière limitative.

### EXEMPLES DE REALISATION DE L'INVENTION

Les exemples suivants concernent la comparaison entre la mise en solution d'une composition A comprenant du percarbonate de sodium, du TAED et du persulfate de potassium, et la mise en solution d'une composition B selon l'invention.

### Exemple 1 (contre-exemple) :

Une solution est préparée à partir de 115 g de la composition A comprenant :
- 100 grammes de percarbonate de sodium,
- 14 grammes de tétra acétyléthylènediamine,
- 1 gramme de persulfate de potassium.
2 à 3 litres d'eau sont ajoutés à la composition A (l'eau peut être déminéralisée ou non).
Le mélange est ensuite agité pour solubiliser les constituants de la composition A.
La quantité d'eau est ensuite ajustée de manière à obtenir une solution de 5 litres.

### Observations :

Le percarbonate de la composition A est rapidement dissout, le TAED restant dans un premier temps à la surface.
Le temps de mise en solution de la composition A (solution aqueuse à 2 % en poids) est de l'ordre de 8 minutes.
Cette solution présente une activité conforme aux normes en vigueur pendant une durée inférieure à 2 jours.

### Exemple 2 (invention) :

Une solution est préparée à partir de 115 g de la composition B comprenant :
- 75.1 grammes de potassium monopersulfate,
- 37.6 grammes de tétra acétyléthylènediamine,
- 2 gramme d'acétate de sodium.
2 à 3 litres d'eau sont ajoutés à la composition B (l'eau peut être déminéralisée ou non).

Le mélange est ensuite agité pour solubiliser les constituants de la composition B.
La quantité d'eau est ensuite ajustée de manière à obtenir une solution de 5 litres.
Le tableau 1 montre le temps de dissolution en fonction de la température de mélange.

**Tableau 1 : Temps de dissolution de la composition B en fonction de la température.**

| Température (°C) | temps de dissolution (minutes) |
|---|---|
| 8 | 28 |
| 15 | 21 |
| 20 | 16 |
| 25 | 12 |
| 30 | 8 |
| 37 | 6 |

Quelle que soit sa température de préparation, cette composition demeure active pendant une période supérieure à 30 jours lorsqu'elle est stockée à la température ambiante.

L'activité de la composition objet de l'invention a été testée sur des virus selon les conditions suivantes :

| Organismes testés | Poliovirus type 1 Adenovirus type 5 |
|---|---|
| Température du test | 20°C |
| Durée de contact | 60 minutes |
| Substance interférente | 0,3 g/l d'albumine sérique bovine |
| Température d'incubation | 37°C |

L'activité virucide de la composition objet de l'invention a été testée sur *Poliovirus type 1* LSc-2ab (Sabin) et *Adenovirus type 5* ATCC VR-5 selon la norme EN 14476:2005+A1:2006.

Les suspensions virales ont été maintenues à une température inférieure à -70°C. Avant utilisation, les cellules ont été multipliées dans une ligne cellulaire HeLa (cellules humaines carinome utérin). Les débris cellulaires ont été enlevés par centrifugation à faible vitesse, et le surnatant contenant le virus a été utilisé pour les tests.

Pour être conforme à la norme EN 14476:2005+A1:2006, la composition testée doit permettre une réduction de 10⁴ (4 log) du nombre d'unités infectieuses.

La composition B (invention) a permis la réduction de 4,00 Log et 4,67 Log sur les virus *Poliovirus type 1* et *Adenovirus type 5* respectivement, après un temps de contact de 60 minutes à 20°C.

## Revendications

1. Composition se présentant sous la forme de poudre, comprenant, en poids sec par rapport au poids de la composition :
- 58.5 à 73.5 % d'au moins un sel de monopersulfate ;
- 25 à 40 % de tétra acétyléthylènediamine ;
- 1.5 à 2.5 % d'au moins un sel d'acétate.

2. Composition selon la revendication 1, ***caractérisée* en ce qu'**elle comprend de 62 à 68 % en poids sec de sel de monopersulfate.

3. Composition selon l'une des revendications précédentes, ***caractérisée* en ce qu'**elle comprend de 30 à 35 % en poids sec de tétra acétyléthylènediamine.

4. Composition selon l'une des revendications précédentes, ***caractérisée* en ce que** le sel d'acétate et l'acétate de sodium.

5. Composition selon l'une des revendications précédentes, ***caractérisée* en ce que** le sel de monopersulfate est le potassium de monopersulfate.

6. Composition selon l'une des revendications précédentes, ***caractérisée* en ce qu'**elle comprend, en poids sec :
- 65.3 % de potassium monopersulfate,
- 32.7 % de tétra acétyléthylènediamine,
- 2 % de sodium acétate.

7. Solution aqueuse comprenant 4500 à 5500 parts en poids d'eau et de 110 à 120 parts en poids de la composition objet de l'une des revendications précédentes.

8. Solution aqueuse biocide selon la revendication 7, ***caractérisée* en ce qu'**elle comprend entre 0.075 et 0.10 mol.L⁻¹ d'acide peracétique.

9. Utilisation de la solution objet de la revendication 7 ou 8, comme agent biocide et/ou virucide et/ou fongicide pour les appareils de dialyse.

10. Procédé de préparation de la solution objet de la revendication 7 ou 8, selon lequel on mélange de 110 à 120 parts en poids de ladite composition avec 4500 à 5500 parts en poids d'eau.
